# EUROPEAN PATENT APPLICATION

(11) **EP 4 480 476 A1**
(43) Date of publication of application: **25.12.2024**
(21) Application number: 24183493.6
(22) Date of filing: 20.06.2024
(51) Int. Cl.: A61K 31/37, A61K 36/185, A61K 31/733, A61K 31/7048, A23L 33/105, A61K 9/16, A61P 39/06, A61P 43/00, A61K 31/718, A61K 33/00

(54) **POWDER AND/OR GRANULAR COMPOSITION BASED ON POMEGRANATE EXTRACT**

(30) Priority: 20.06.2023 IT 202300012771
(71) Applicant: Unifarco S.p.A., 32035 Santa Giustina (BL) (IT)
(72) Inventor: Baratto, Giovanni, 32035 Santa Giustina BL (IT); Ferrari, Sara, 32035 Santa Giustina BL (IT); Casanova, Elena, 32035 Santa Giustina BL (IT); Francescato, Stefano, 32035 Santa Giustina BL (IT)
(74) Representative: Perani & Partners S.p.A.

(57) **Abstract**

Powder and/or granular composition A) based on pomegranate extract comprising:
a) a dried pomegranate extract comprising punicalagin A and B, ellagic acid and/or derivatives thereof;
b) a powder comprising or consisting of Inulin
wherein:
-the content in total punicalagin in A and B, ellagic acid and/or derivatives thereof is comprised between 4 and 15% by weight on the total weight of said powder and/or granular composition.
- the weight ratio between dried pomegranate extract / inulin is comprised between 1: 1 and 3: 1.

This powder and/or granular composition is used to prevent damage from ageing, to improve intestinal homeostasis and/or to promote the metabolism of polyphenols by the intestinal microbiota.

## Description

### Field of the invention

The present invention relates to a powder and/or granular composition based on pomegranate extract, relative oral formulations comprising said solid and/or granular compositions as a supplement or in nutraceutical product, and the use of said powder and/or granular compositions in the prevention of ageing damage, to improve intestinal homeostasis and/or to promote intestinal microbiota metabolism.

### Prior art

Research on the subject of ageing and longevity explores the decline of body functions during adulthood: in 2023, twelve hallmarks were proposed which are intrinsically linked to each other and are not independent of each other [Cell. 2023 Jan 19;186(2):243-278]*.*

Such a classification is inevitably arbitrary, but three criteria have been proposed that must be applied for each hallmark of ageing:
(1) the manifestation of alterations accompanying the ageing process is time-dependent,
(2) ageing can be experimentally accelerated by accentuating the hallmark,
(3) it is possible to slow, stop or reverse ageing by means of therapeutic interventions on the hallmark.

The interdependence of the hallmarks of ageing means that the experimental accentuation or attenuation of a specific hallmark usually affects other hallmarks as well. This underlines the fact that ageing is a complex process that must be conceived as a whole. As a result, each of the hallmarks should be considered an entry point for future exploration of the ageing process, as well as for the development of new anti-ageing and pro-longevity drugs.

The twelve hallmarks of ageing proposed by López-Otin and colleagues are: genomic instability, telomere attrition, epigenetic alterations, loss of proteostasis, disabled macroautophagy, deregulated nutrient-sensing, mitochondrial dysfunction, cellular senescence, stem cell exhaustion, altered intercellular communication, chronic inflammation, and dysbiosis. These hallmarks are grouped into three categories: primary, antagonistic, and integrative.

Mitochondria are ovoid-shaped organelles present in all cells, separated from the cellular cytoplasm by two membranes, one external and one internal. Between the two we find an intermembrane space, while the internal membrane encloses the mitochondrial matrix. Mitochondria are often referred to as the "powerhouses" of the body. In fact, those biochemical processes (Krebs cycle and oxidative phosphorylation) take place therein, which allow energy to be extracted from organic substrates to produce an electrochemical gradient, which is exploited to synthesize adenosine triphosphate (ATP). ROSs (i.e., oxygen compounds with high oxidizing activity) produced endogenously, exposure to exogenous agents (environmental toxins, smoke...) and even ageing, can cause mitochondrial dysfunction, which is closely related to inflammation through a series of mechanisms. Initially, the accumulation of ROS results in increased mitochondrial permeability, with the exit of mitochondrial components in the cytoplasm. Such components are recognized as potential hazards by specific cytoplasmic receptors (PRRs = pattern recognition receptors) and a complex, called the inflammasome, is formed. In turn, the inflammasome activates the inflammatory cytokine IL1β, which then recruits other components of the immune system to destroy the "infected" cell, thus leading to cell destruction.

As mentioned, ageing is associated with a decrease in mitochondrial efficiency and a consequent increase in free radicals.

In addition to energy production and the regulation of the redox state of the cell, the mitochondria are also involved in other fundamental cellular processes, including apoptosis, cell cycle regulation, heme synthesis, cholesterol synthesis, heat production, β-oxidation of fatty acids and also has a function of depositing Ca2+ ions in the mitochondrial matrix. [Life Extension Foundation for Longer Life. Inflammation (Chronic). Disease Prevention and Treatment. Fifth Edition, p. 795-807]

Therefore, maintaining mitochondrial function is essential during development and throughout life.

It has been shown that with ageing, mitochondrial turnover also slows down and that this contributes to the pathogenesis of age-related diseases. Calorie restriction (CR) is a well-known intervention that has been shown to improve mitochondrial biogenesis (mitogenesis) and mitophagy (i.e., elimination of damaged mitochondria), thereby attenuating age-related decline in mitochondrial function. However, strict and lifelong compliance with this diet is complex and this has promoted the search for compounds that can have "calorie restriction mimetic" functions. In particular, plant polyphenols have been shown to have interesting properties, as they can partly mimic the beneficial effects of calorie restriction, inducing molecular mechanisms governing mitochondrial biogenesis and mitophagy. [Polyphenols as Caloric Restriction Mimetics Regulating Mitochondrial Biogenesis and Mitophagy. Davinelli, S. et al. 2020, Trends in Endocrinology&Metabolism]

Pomegranate (scientific name *Punica granatum* (L., 1753)) is a plant belonging to the Lythraceae family (formerly in Punicaceae). It is a shrub or a small deciduous fruit tree that reaches dimensions between five and eight metres in height. The name Pomegranate comes from the medieval Latin *Pomum* "apple" and *granatum* "with seeds". The generic name Punica refers to the Phoenicians, who contributed to the spread and cultivation of this species.

The pomegranate is native to the region of Persia (modern-day Iraq) and the western Himalayan chain. It has been cultivated for several millennia in Iran, Iraq, Azerbaijan, Afghanistan, Pakistan, India, Russia and the Mediterranean region.

In the Indian subcontinent, *Punica granatum* has been used for several thousand years in Ayurvedic medicine. The rind of the fruit and the bark of the pomegranate are used as a traditional remedy against diarrhoea, dysentery and intestinal parasites.

There are over 1,000 cultivars of *Punica granatum* L., native to the Middle East, spread throughout the Mediterranean, eastward to China and India, and in South-west America, California and Mexico and the New World.

This species has elongated leaves arranged alternating. The flowers are bright red, orange or white and can be grouped or scattered sporadically near the end of the branches. The fruits are hard berries with a round leathery skin, when ripe they grow up to 5-12 cm in diameter depending on the variety.

The seeds are covered with a fleshy aril that constitutes the edible part of the fruit; their colour varies from red to pinkish white. [Punica granatum L. (Punicaceae): Lifeline for Modern Pharmaceutical Research. Mehta, D e Mehta, M. s.l. : Inventi Impact: Ethnopharmacology, 2012*.]*

Pomegranates are an important source of bioactive compounds such as phenols, flavonoids, ellagitannins, in particular punicalagin, which activate both the AMPK pathway and also phase II enzymes and induce mitochondrial biogenesis.

An increasing number of work has shown that the polyphenolic compounds of the pomegranate are able to act as modulators of mitophagy in experimental models.

Urolithin A (UA) is a natural compound produced by intestinal bacteria from ellagitannins (ETs) and ellagic acid (EA): complex polyphenols abundant in foods such as pomegranates, berries and nuts. Urolithin A was discovered 40 years ago, but its impact on the ageing process only been explored recently: it improves cellular health by increasing mitophagy and mitochondrial function and reducing harmful inflammation. Several preclinical studies show how Urolithin A protects against ageing and age-related conditions affecting the muscles, brain, joints, and other organs. In rodents, Urolithin A increases mitophagy in muscle cells in a dose-dependent manner, and this was seen to lead to improved muscle function and energy efficiency during endurance efforts; this effect is independent of age and diet [Urolithin A induces mitoophag and prolongs lifespan in C. elegans and increases muscle function in rodents. Ryu, D. et al. S.l. : Nature Medicine, 2016].

In addition to the positive effect on mitochondrial health, duration and muscle efficiency, in the preclinical models analysed it was also seen that Urolithin A supplementation was able to reduce the levels of inflammaging, a process that contributes to age-related decline. The mechanism of action of Urolithin A in the context of inflammation is actually not yet fully elucidated and seems to vary in different tissues or under different conditions.

The health benefits of Urolithin A have been described in a wide range of *in vivo* models of health decline related to both ageing and chronic disease [Impact of the Natural Compound Urolithin A on Health, Disease, and Aging. D'Amico, D. et al. s.l.: Trends in Molecular Medicine, 2021].

Preclinical *in vivo* studies and clinical trials have highlighted some of the key biological effects on different tissues and organs including:
- Muscle: increased muscle strength and endurance, decreased age-related muscle dysfunction;
- Heart: reduction of inflammatory parameters, protection from atherosclerosis, improvement of contractility and myocardial dysfunctions;
- Brain: memory improvement in mice with overexpression of β-amyloid protein, reduction of inflammation in the brain;
- Joints: decrease in joint degeneration;
- Immune system: decrease of proinflammatory cytokines and neuroinflammation;
- Intestine: protective effect in IBD models, with reduction of inflammatory markers and protective action on the intestinal mucosa;
- Liver and pancreas: reduction of triglyceride accumulation in the liver, reduction of cholesterol and improvement of insulin sensitivity in induced animal obesity model;
- Kidney: reduction of cisplatin-induced tubular damage in animal models of acute renal injury.

It is important to underline that Urolithin A thus safeguards against physiological decline, as illustrated by the improvement of muscle function in young animals and the prevention of age-related muscle decline in elderly mice.

However, the conversion of precursors (Ellagitannins, Ellagic Acid), taken with the diet, into Urolithin A does not occur in all individuals in the same way. The main determinant is the type of microbiota: it has been seen that this conversion process occurs only in about 40% of elderly subjects. [Impact of the Natural Compound Urolithin A on Health, Disease, and Aging. D'Amico, D. et al. S.l. : Trends in Molecular Medicine, 2021].

To overcome the failure to convert precursors to Urolithin A, a recent study found that direct supplementation with pure Urolithin A is able to increase endogenous levels thereof, thus overcoming differences in microbiota and diet [Direct supplementation with Urolithin A overcomes limitations of dietary exposure and gut microbiome variability in healthy adults to achieve consistent levels across the population. Singh, A. et al. s.l. : European Journal of Clinical Nutrition, 2022].

### Summary

The applicant has developed a powder and/or granular composition A) based on pomegranate extract comprising:
a) a dried pomegranate extract comprising punicalagin A and B, ellagic acid and/or derivatives thereof;
b) a powder comprising or consisting of Inulin
   and which is characterized in that:
   - the content in total punicalagin in A and B, ellagic acid and/or derivatives thereof is comprised between 4 and 15% by weight on the total weight of said powder and/or granular composition.
   - the weight ratio between dried pomegranate extract / inulin is comprised between 1: 1 and 3.

In fact, the applicant has found the inulin contained therein, in addition to constituting a technological excipient, also acts as an active ingredient promoting the transformation of punicalagin into Urolithin A and is also able to act directly on mitochondrial metabolism.

### Figures

Figure 1 shows the processing diagram of a pomegranate batch with the yields of each process disclosed in detail in the examples in step 1
Figure 2 shows in detail the biomass compositions of a processing batch
Figure 3 shows the extraction yields of two biomasses: Pericarp and whole fruit, under different extraction conditions.
Figure 4 shows the quantification of the active ingredients forming the phytocomplex extracted from two biomasses: pericarp and whole fruit.
Figure 5 shows the results related to the conversion and relative consumption of ellagic acid in faecal samples from 8 subjects in the experiment disclosed in Example 2
Figure 6 shows the results related to the conversion of urolithin A in faecal samples from 8 subjects in the experiment disclosed in Example 2.
Figure 7-A) and B) summarizes the results obtained in 6/8 patients reported in figures 5 and 6 responding to the treatment with pomegranate extracts.

### Detailed description of the invention

For the purposes of the present patent application, the definitions "comprising" and "containing" provide for the possibility of further components in addition to those mentioned after said definition.

Conversely, for the purposes of the present invention the definition "consisting of" excludes the possibility of further components other than those listed after said definition.

For the purposes of the present invention, a dry extract is intended as a solid preparation obtained by extraction and subsequent evaporation of the solvent used during extraction. Dry extract also means an extract having a dry residue not less than 90% by mass.

Soft extract means a preparation with an intermediate consistency between fluid extracts and dry extracts, obtained by evaporation of the solvent used for their preparation and normally characterized by a dry residue not less than 25% by mass.

"Phytocomplex" means, for the purposes of the present invention, the set of molecules directly extracted from biomass of plant origin. The known therapeutic action, exerted by biomass of plant origin, is given by the sum of the effects of the active ingredients or molecules forming the phytocomplex.

For the purposes of the present invention, the definition of ellagic acid derivatives means: phenolic and glycosidic derivatives of ellagic acid.

It is known from the literature that a pomegranate extract has a low bioavailability in vivo because not all individuals are able to form urolithins after ingesting ellagitannins (punicalagin) and ellagic acid, regardless of the amount ingested.

Medium molecular weight polyphenols, such as punicalagin, are not absorbed in the stomach and small intestine, reaching the colon almost unchanged, causing a modulation of the composition of the intestinal microbiota, a population of microorganisms beneficial for humans.

The powder and/or granular composition A) which is the object of the present invention is in the form of a powder when the particle size distribution D50 is less than 250 µm.

The powder and/or granular composition A) which is the object of the invention is in granular form when the particle size distribution is comprised between: 250 µm and 2 mm.

In the powder and/or granular composition A) according to the present invention, in addition to inulin, the powder b) can comprise maltodextrin and/or silica.

In particular, the concentration of the maltodextrin in the composition A) is comprised in the powder b) in concentrations comprised between 0 and 40% by weight on the total weight of said powder and/or granular composition A), while the silica in the powder b) is comprised between 0 and 6% by weight on the total weight of said final composition A).

If maltodextrin and/or silica is present together with the inulin, the concentration of the latter within the final composition is present in amounts comprised between 20 and 30% by weight on the total weight of said composition A).

In the composition according to the present invention the dry pomegranate extract is selected from pericarp, endocarp, seeds, whole fruits, juice or mixtures thereof, preferably it is pericarp extract.

For the purposes of the present invention, in the present disclosure the aforesaid parts of the pomegranate are also defined biomass.

Preferably the content of total punicalagin A and B, ellagic acid and/or derivatives thereof is comprised between 4 and 10%, more preferably between 6 and 8% and according to a preferred embodiment of the invention it is 7% by weight on the total weight of said composition A).

Preferably in the compositions A) according to the present invention, the dry pomegranate extract a) /inulin weight ratio is comprised between 1.5:1 and 2.5: more preferably between 1.8:1 and 2.0:1.

Preferably the compositions A) according to the present invention contain the dry extract a) in amounts comprised between 45 and 50% by weight, more preferably 49% by weight, the maltodextrin in amounts comprised between 21 and 25% by weight, more preferably 22% by weight, the inulin between 23 and 28% by weight, more preferably 25% by weight, the silica in amounts comprised between 2 and 6% by weight, more preferably 4% by weight, on the total weight of the powder and/or granular composition A).

The composition in powder or granular form A) is obtained by drying a mixture A-1) comprising:
a-1) a soft extract of pomegranate; and
b) a powder comprising or consisting of inulin.

Preferably when it is desired to obtain a powder composition, the drying is obtained by the following process comprising the following steps:
I) provision of a soft extract a-1);
II) preparation of an aqueous suspension consisting of the soft extract a-1) and of the powder mixture b) comprising or consisting of inulin:
III) drying of the aqueous suspension, by evaporation of the solvent.

When the composition A) is in granular form, it is obtained by a process comprising the following steps:
i) the soft pomegranate extract a-1) is prepared;
ii) the powder b) comprising or consisting of inulin is poured into a fluid bed granulator and mixed;
iii) in said fluid bed granulator, the soft extract a-1) prepared in step i) is granulated on the powder b) comprising or consisting of inulin prepared in the previous step ii);
iv) the granulate obtained in step iii) is discharged.

In both processes, the first step of preparing the soft extract a-1) comprises the following steps:
1. provision of at least one fresh and/or dried part e) of the pomegranate selected from pericarp, endocarp, seeds, whole fruit and juice;
2. mixing at least a part e) of the pomegranate with a hydroalcoholic solvent f), preferably ethanol in water at concentrations comprised between 50% and 70% w/w, in which the weight ratio e):f) is comprised in the range from 1:1 to 1:25, preferably from 1:3 to 1:20, more preferably from 1:6 to 1:15, said mixing being followed by a heating under stirring at a temperature comprised in the range from 30°C to 90°C, preferably from 40°C to 80°C, more preferably from 50°C to 70°C
3. extraction of the suspension from step 2) until complete exhaustion of the biomass, preferably with ultrasound or hot mixing at a temperature comprised between 50°C and 70°C
4. separation of the solid material from the liquid phase by at least one filtration or centrifugation;
5. concentration by vacuum evaporation of the filtered liquid coming from step 4) until obtaining a soft extract having a concentration of dry material in water comprised between 25% and 75%.

The object of the present invention is a suitable oral formulation comprising the composition A) which is the object of the invention in combination with suitable excipients and/or diluents.

This oral formulation is in particular used as a supplement or as a nutraceutical product.

For the purposes of the present invention, "nutraceutical" is intended as the definition given by Stephan De Felice in 1989 or a food (or part thereof), preferably a part thereof (specifically the extract which is the object of the present invention) and which has positive effects on well-being and health, including the prevention and treatment of diseases.

For the purposes of the present invention, food supplement means a formulation which falls under the definition of Directive 2002/46/EC and subsequent amendments. In this legislation, food supplements are precisely defined as: "foodstuffs the purpose of which is to supplement the common diet and which are a concentrated source of nutrients, such as vitamins and minerals, or other substances with a nutritional or physiological effect, in particular, but not exclusively, amino acids, essential fatty acids, fibres and extracts of plant origin, both mono- and multi-compounds, in pre-dosed forms".

For the purposes of the present invention, suitable excipients and/or diluents of such oral compositions are intended as pharmaceutical and/or food grade substances, optionally present in the formulations for oral use of the invention, such substances being devoid of therapeutic activity suitable for pharmaceutical or food use. Acceptable excipients for pharmaceutical or food use are all those substances commonly known to the person skilled in the art for the preparation of oral forms such as powders, granules, capsules, tablets, solutions, oral suspensions. By way of nonlimiting example, such excipients can be selected from the list consisting of
a) Diluents such as dibasic calcium phosphate, microcrystalline cellulose and cellulose derivatives
b) Thickeners such as hydroxypropylmethylcellulose and cellulose derivatives, gums
c) Sweeteners such as sucralose, sorbitol, mannitol and other polyols
d) Lubricants such as magnesium stearate, waxes, stearic acid,
e) Dispersants
f) Flavourings
g) Adsorbents such as silicon dioxide, talc, starch
h) Glidants
i) Non-stick agents such as talc, colloidal silica, corn starch, silicon dioxide
j) Dyes such as iron oxides, riboflavin, chlorophyll, etc.
k) Antioxidants
l) Binders such as starch, gelatin gums, sodium alginate, cellulose derivatives
m) Disintegrants such as microcrystalline cellulose, starch, crospovidone, alginic acid
n) Plasticizers such as ethylcellulose and cellulose derivatives, glycerol and sorbitol
o) Preservatives such as potassium sorbate and sodium benzoate
p) Viscosifiers
q) Emulsifiers
r) Humectants) Solvents (including water, ethyl alcohol)

In an embodiment, the oral formulation comprising the powder and/or granular composition A) according to the present invention, comprising the pomegranate extract and at least one food or pharmaceutical grade additive and/or excipient, as defined in the present disclosure, can comprise a further component (or active component) selected from the group comprising or, alternatively, consisting of: manganese, purified fish oil, quercetin, zinc, pterostilbene, copper, citicoline, resveratrol, carnosine, L-carnitine, hydroxymethylbutyrate, Coenzyme Q10, Vitamins of group B, C, D, E, Organic and/or inorganic salts of magnesium, of selenium.

The composition which is the object of the invention can be used in the prevention of damage from ageing, in particular when said damage involves different tissues and organs such as muscles, the heart, brain, joints, immune system, liver, pancreas and kidney. Furthermore, the same composition in powder and/or granular form A) according to the present invention is used to improve intestinal homeostasis and to promote the metabolism of polyphenols by the intestinal microbiota.

### EXAMPLES

Several tests, divisible into phases, or successive steps, have been carried out, which led the applicant to identify the composition in powder or granular form A) which is the object of the present invention, a solution to the technical problem of the prior art.

The steps are disclosed in detail below, with details of some experiments for exemplary but not exhaustive purposes.

### STEP 1: BIOMASS PREPARATION AND CHARACTERIZATION

In the first step, the research work served to identify the yields, in terms of biomass, and the compositions of the different parts of the pomegranate or biomass. For the purposes of the present invention, the term "Biomass" is intended as the starting plant material (Whole fruit) and also the parts which compose it (Pericarp, Juice, Endocarp, Seeds).

Below is a brief disclosure of the processing process which led to obtaining each biomass used for the extraction of STEP 2.

### STEP 1.A: Division of Pericarp into Endocarp + seeds

The whole fruit is divided manually, taking care to remove the pericarp from the endocarp and the seeds.

After dividing the two biomasses, proceed to:
- for the Pericarp, a drying process in an air dryer at 40°C at a final humidity of less than 10% for 48 hours
- for the Endocarpo, plus seeds, proceed with a pressing by means of a screw press and storage of the juice obtained
- A drying process is applied to the squeezed biomass of Endocarp + seeds in an air dryer at 40°C at a final humidity less than 10% for 48 hours

### STEP 1.B: Processing the whole fruit

- The whole fruit is pressed using a screw press and the juice obtained is stored
- A drying process is applied to the squeezed biomass of the whole fruit in an air dryer at 40°C at a final humidity less than 10% for 48 hours

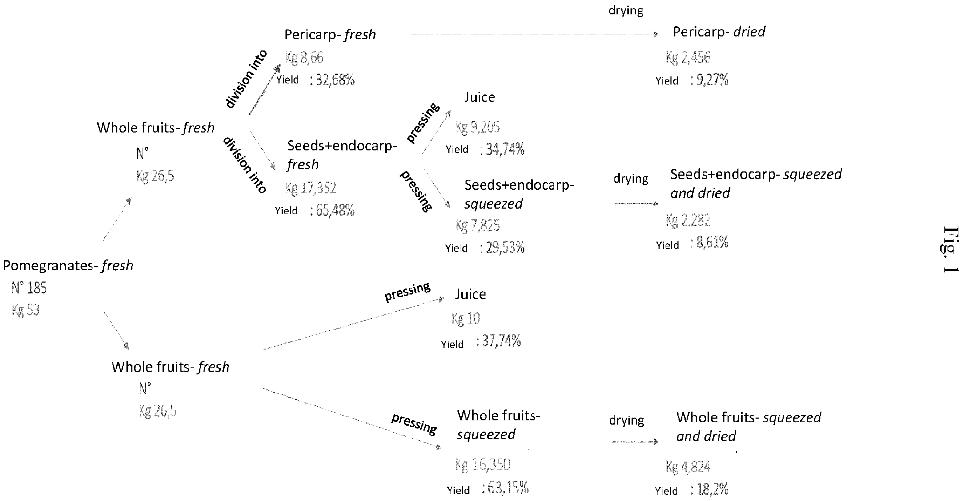

The yields of each process are schematically shown in Figure 1.

The starting biomasses are therefore: pericarp, endocarp, seeds, whole fruit, juice.

A first characterization was carried out on these biomasses by quantifying some components of interest, such as: Punicalagin, Ellagic acid derivatives, Anthocyanins, Medium Molecular Weight Polysaccharides (1-10kDa).

The titration of the content in total punicalagin A and Band ellagic acid and derivatives thereof was carried out in HPLC-DAD, mobile phase water 0.1% formic acid and MeOH. Stationary phase: phenomenex Synergy column 4 µm Max-RP 80a 3x150 mm. The flow was maintained at 0.4 ml/minute. The reading was monitored at 355 nm.

The gradient is shown in the following table 1:

**Table 1 Elution gradient in HPLC-DAD**

| **Tempo** | | **MeOH (%)** | **Acqua 0.1% acido formico (%)** | |
|---|---|---|---|---|
| | 0 | 5 | | 95 |
| | 1.5 | 5 | | 95 |
| | 15 | 85 | | 15 |
| | 20 | 100 | | 0 |
| | 26 | 100 | | 0 |
| | 26.5 | 5 | | 95 |
| | 30 | 5 | | 95 |

Reference standard: Punicalagin A+B and ellagic acid mixture.

Polysaccharide quantification was performed by Analytical Method carried out in HPLC-ELSD-SEC (Size exclusion chromatography). HPLC-SEC analysis allows polysaccharides to be separated according to their molecular weight: low-medium molecular weight compounds (glucose, dextran 1000) will have longer retention times, as they remain in the column longer with respect to the higher molecular weight polysaccharides (>5000 Da). A PolySep-GFC-P 3000 300x7.8 mm column with Guard Column 5x7.8 mm pre-column was used as the stationary phase, while 0.1M ammonium acetate buffer, isocratic for 20 minutes, was used as the mobile phases. An Evaporative Light Scattering Detector (ELSD) Sedex 60 LT. was used as detector.

An example of the composition of some processed biomasses is shown in table 2.

**Table 2 Quantification of polysaccharides contained in the biomass processed in step 1**

| **Campione** | % composti a medio PM (espressi come destrano 1000 Da) | % composti a medio-alto PM (espressi come destrano 12000 Da) |
|---|---|---|
| Frutto intero | 6.17±0.50 | - |
| Endocarpo+semi | 6.65±0.11 | - |
| Pericarpo | 7.04±0.02 | - |

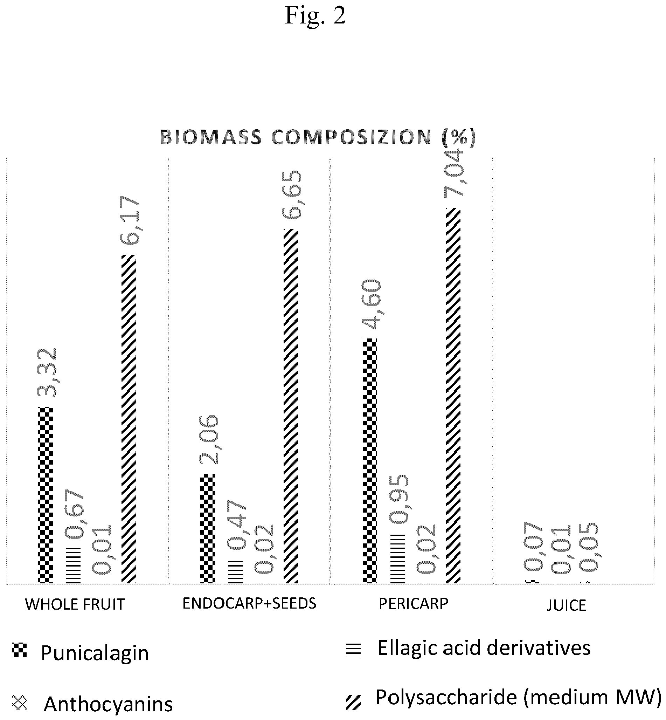

The biomass compositions of a processing batch are shown in detail in Figure 2.

### STEP 2: DEVELOPMENT OF THE BIOMASS EXTRACTION METHOD

After the preparation and characterization of the biomasses, some extraction tests were developed, testing different conditions in terms of solvent, solvent concentrations and extraction temperatures.

In general, the extraction method consists of:
- Placing 20 g of finely crushed plant material in 200 ml of solvent and sonicating for 30 minutes in an ultrasonic bath. Such an extraction cycle is repeated several times until the biomass is exhausted (HPLC -DAD analysis with the above method of the content of total punicalagin A+B and ellagic acid and derivatives in the exhausted biomass)
- The plant material is separated from the solvent by gauze filtration and second vacuum filtration with Buchner funnel (11 micron porosity filter).
- The extract is returned to dryness by a rotary evaporator at a temperature of 40°C-70°C.
- Water (room temperature (RT), equal to 25°C and 70°C)
- 20% Ethanol solution (ethanol:water mixture = 20:80) (RT and 50°C)
- 50% Ethanol solution (ethanol:water mixture = 50:50) (RT and 50°C)
- 70% Ethanol solution (ethanol:water mixture = 70:30) (RT and 50°C)

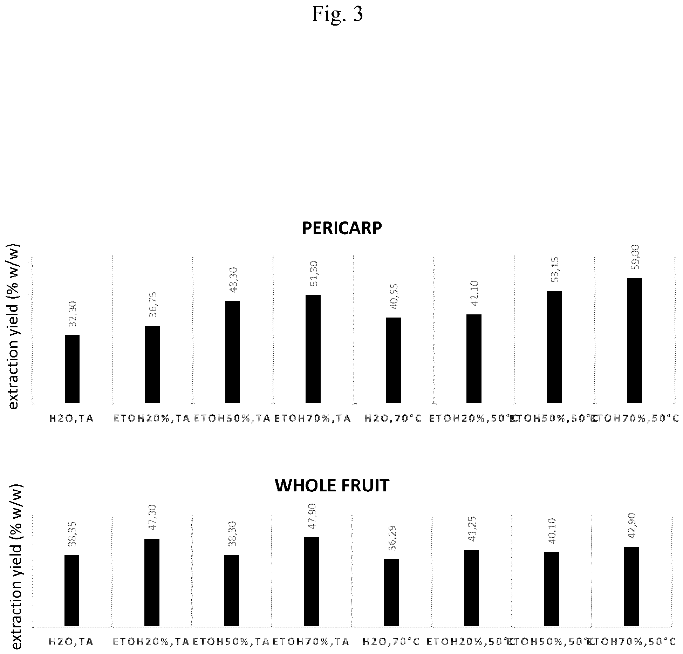

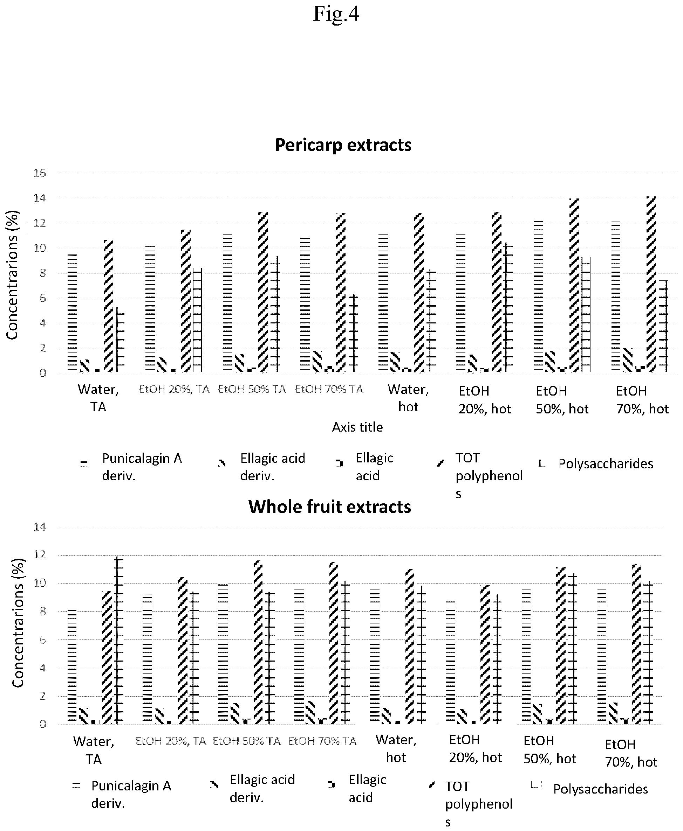

Figure 3 shows the extraction yields of two biomasses: Pericarp and Whole fruit, under the different conditions listed above. Figure 4 shows the titres of the active ingredients forming the phytocomplex extracted from each biomass. From this step it was possible to define that the solvent conditions/mixtures that allow to further concentrate the fraction of the extract containing punicalagin A and B and ellagic acid and derivatives thereof are:
- 50% w/w Ethanol solution (ethanol: water mixture =50:50) at 50°C
- 70% w/w Ethanol solution (ethanol: water mixture =70:30) at 50°C

### STEP 3: SELECTION OF EXCIPIENTS TO SUPPORT POMEGRANATE EXTRACT

In this step the Applicant carried out tests to identify the most suitable excipients for formulating a dry extract comprising the pomegranate extract so as to obtain a dry extract.
- stable (chemically, physically and organoleptically),
- effective (able to exert a biological and/or molecular action by acting on the in vivo increase of bioavailability of the phytocomplex extracted from pomegranate thanks to the presence of inulin)
- with technological features suitable for inclusion in a solid or liquid nutraceutical formulation (flowability, particle size, density, compressibility, etc.).

Due to the natural variability of the biomass from which they are obtained, extracts of plant origin may have differences in the concentration of the phytocomplex, which therefore needs to be standardized.

The technological supports normally used to stabilize and standardize extracts of plant origin are, to name a few: maltodextrins, silicon dioxide, gum arabic and sugars of various kinds.

Maltodextrin is a polysaccharide commonly used in various fields, including medical and food. In plant extracts, maltodextrin plays several important roles in improving product efficacy and palatability. Firstly, maltodextrin can improve the solubility of ingredients in plant extracts. Some active ingredients may not be soluble in water or other solvents, making them difficult to solubilize. Maltodextrin can increase water solubility, facilitating the dissolution of these active ingredients and improving their extraction efficiency.

Secondly, maltodextrin may improve the stability of plant extracts. During storage, transportation and processing, plant extracts may be subject to degradation or deterioration: maltodextrin has good stability properties that can protect the active ingredients in plant extracts and prolong the duration thereof.

Thirdly, maltodextrin may promote the absorption of the active ingredients in plant extracts. In the intestinal tract, maltodextrins can form a protective film that protects the active ingredients from the corrosive effects of gastric acid and bile, improving the bioavailability thereof and facilitating the absorption thereof.

Lastly, maltodextrin can reduce bitterness and unpleasant tastes in plant extracts. Overall, maltodextrin is an important ingredient in plant extracts because it improves the solubility, stability and bioavailability of the active ingredients and improves the palatability thereof.

Silicon dioxide (Silica) is a powder that in pharmaceutical technology is a glidant and adsorbent in the preparation of solid forms.

To support the efficacy part, the applicant also included Inulin in the excipients, which is a prebiotic recovered from different plant sources, used for its healthy properties. Following are some examples of laboratory tests that were necessary to identify the right composition of the granulate comprising soft pomegranate extract, as well as the proportion between the individual components.

### Step 1 - Step 3

120 g of pomegranate extract (pericarp) extracted in 70% Ethanol at 50°C, was concentrated in a rotary evaporator under vacuum until the ethanol was completely removed (measurement by alcohol meter). A mixture of excipients was added to the soft concentrated extract thus obtained, as shown in table 3.

The concentration of the solid residue (extract + mix of excipients) in aqueous suspension is approximately 15-20%. Such a suspension is dried using a spray drying technique, using a BUCHI B-290 instrument with an inlet temperature of 60°C, outlet of 120°C, 20% pump, suction flow 38mL/min and 100% suction.

**Table 3 Composition of extracts with excipients**

| **EXTRACT COMPOSITION** | |
|---|---|
| extract no. 208 | Extract 70%, Inulin 30% |
| extract no. 209 | Extract 100% |
| extract no. 210 | Extract 70%, Silicon dioxide 1%, Maltodextrins 4%, Inulin 25% |

Table 4 shows the titres of the extracts disclosed in table 3.

**Table 4 Extract titres expressed as a total percentage of polyphenols, expressed as the sum of punicalagin A and B and ellagic acid and derivatives thereof.**

| **Campione** | **% polifenoli** | | |
|---|---|---|---|
| | Espressi come punicalagina A | Espressi come acido ellagico | **TOT** |
| | | | |
| Estratto N.208 | 7.83 | 1.80 | **9.63** |
| Estratto N.209 | 10.46 | 1.05 | **12.51** |
| Estratto N.210 | 7.44 | 1.62 | **9.07** |

### STEP 4: POMEGRANATE EXTRACT GRANULATION TESTS WITH MALTODEXTRINS, INULIN AND SILICA

The excipient drying process disclosed in step 3 was also carried out by means of a fluid-bed granulation process to obtain a stable final dry extract. For this reason, the Applicant, keeping the excipients selected in step 3, set up a fluid-bed granulation process to obtain a stable final dry extract.

The process for preparing the granulate comprises the following steps:
i) the soft pomegranate extract is prepared;
ii) the maltodextrins, inulin and silica are mixed in a fluid bed granulator;
iii) the soft extract prepared in step i) is sprayed in said fluid bed on the mixture obtained in step ii)
iv) the granulate obtained is unloaded.

To obtain the preferred mixture of extract and excipients, several tests were carried out on different compositions shown in the following tables.

### STEP 4 - TEST 1:

| | **Kg** | **Kg** | **%** |
|---|---|---|---|
| Extract | 12.25 (soft extract) | 6.37 (dry extract) | 37.7 |
| Juice | 10 | 1.61 | 9.5 |
| Inulin | | 6 | 35.5 |
| Maltodextrins | | 2.5 | 14.8 |
| Silica | | 0.4 | 2.4 |
| Total | | 16.88 | 100 |
| | Actual titre¹ | 6.640 | |
| | LOD² | 5.944 | |
| | aw³ | 0.327 | |

In this test we also used the juice (whole fruit), but the presence of sugars in this biomass results in the formation of a very hygroscopic extract, therefore, it is unstable and compacts.

### STEP 4 - TEST 2

| | **Kg** | **Kg** | **%** |
|---|---|---|---|
| Extract | 5.125 (soft extract) | 2.665 (dry extract) | 45.6 |
| Juice | | 0 | 0 |
| Inulin | | 1.6 | 27.2 |
| Maltodextrins | | 1.4 | 24 |
| Silica | | 0.2 | 3.3 |
| Total | | 5.85 | 100 |
| | Actual titre (%) | 6.680 | |
| | LOD | 6.364 | |
| | aw | 0.272 | |
| ¹ % w/w of punicalagin A and B and ellagic acid and/or derivatives thereof on the total weight of the granulate | | | |
| ² LOD % (Loss on drying) | | | |
| ³ aw (Water activity) | | | |

This extract was found to be suitable for the purposes set out by the applicant; however, an additional test was carried out to evaluate the possibility of increasing the percentage of soft pomegranate extract in the final granulate.

### STEP 4 - TEST 3

| | **Kg** | **Kg** | **%** |
|---|---|---|---|
| Extract | 6.125 (soft extract) | 3.185 (dry extract) | 49.1 |
| Juice | | 0 | 0 |
| Inulin | | 1.6 | 24.67 |
| Maltodextrins | | 1.4 | 21.59 |
| Silica | | 0.3 | 4.63 |
| Total | | 6.485 | 100 |
| | Actual titre % | 7.160 | |
| | LOD | 6.139 | |
| | aw | 0.227 | |

**The Extract of Step 4** - **test 3 was found to be stable.** Compared to test 3, in order to stabilize the granulate which in this test contains a higher amount of soft pomegranate extract, it was necessary to increase the percentage of silica.

With respect to commercially available pomegranate extracts, the powder and/or granular composition A) developed by the Applicant is less pure and less concentrated in terms of phytocomplex actives (e.g., total percentage of polyphenols), however the phytocomplex extract, according to the methods disclosed in step 2, despite being more heterogeneous than other pomegranate extracts of the prior art, the Applicant has found this heterogeneity to be useful for having a greater action on the intestinal microbiota. In general, the percentage of maltodextrin may vary because it is an inert excipient useful for standardizing the titre of pomegranate soft extract in punicalagin A and B and ellagic acid and derivatives thereof, to the desired percentage, for example 7%.

(The starting biomass always has a variable titre, and in fact before the actual drying process, the soft extract is titrated in terms of punicalagin A and B and ellagic acid and derivatives thereof).

### EXAMPLE 2

In-vitro tests were carried out according to the "Colon on a plate^{®}" technology⁴, applying to faecal inocula of 8 subjects:
1. maltodextrin + inulin + silica (Negative control) at a concentration of 1.78 g/l.
2. a dry extract of pomegranate without excipients titrated in punicalagin and ellagic acid (a commercial extract can be used, for example POMELLA^{®}): at a concentration of 0.31 g/l (Positive control)
3. Pg extract of the invention, concentration 3.5 g/l, containing inulin, maltodextrin and silica

The concentration selected chosen for both the Pg extract and the commercial extract was obtained by titration of both extracts in both punicalagin and ellagic acid, according to the following protocol.

### Sample preparation and quantification

40 mg of pomegranate rind powder was weighed and extracted in 10 ml of methanol:water 80:20. The samples were extracted in ultrasonic bath for 30 minutes, centrifuged at 13,000 rpm for 10 minutes and placed in vials for analysis.

**Methods:** HPLC-DAD, mobile phase water 0.1% formic acid and MeOH. Stationary phase: Phenomenex Synergi 4 µm Max-RP 80A 3x150 mm column.

The flow was maintained at 0.4 ml/minute. The reading was monitored at 355 nm. The gradient used is shown in the table.

| **Time** | **MeOH (%)** | **Water 0.1% formic acid (%)** |
|---|---|---|
| 0 | 5 | 95 |
| 1.5 | 5 | 95 |
| 15 | 85 | 15 |
| 20 | 100 | 0 |
| 26 | 100 | 0 |
| 26.5 | 5 | 95 |
| 30 | 5 | 95 |
| ⁴ 48 hours anaerobic incubation at 37°C faecal inoculum in carbohydrate depleted medium | | |

The following analytical standards were used as reference:

### Punicalagin A+B Mixture

- supplier: Cayman Chemical.
- solvent: methanol:water 80:20
- concentration range for calibration line: 107-10.7 µg/ml (10-5-1 µL injection)

### Ellagic acid:

- supplier: Sigma-Aldrich.
- Solvent: methanol
- Concentration range for calibration line: 70-7 µg/ml (10-5-1 µL injection)

The samples were identified and quantified with UV spectroscopy.

There are peaks from 2 to 10 minutes with UV spectra compatible with punicalagin and in the quantification they are expressed as punicalagin A+B.

From 10 minutes onwards, there are peaks with UV spectra compatible with ellagic acid and derivatives and in the quantification they are expressed as ellagic acid.

The results of the titration are as follows

**Table**

| SAMPLE | Punicalagin A+B % (w/w) on the total weight of the extract | Ellagic Acid | Total punicalagin + Ellagic acid |
|---|---|---|---|
| Sample 1 Dry pomegranate extract according to invention (pg) | 2.88 | 3.89 | 6.77 |
| Dried pomegranate extract without excipients | 29.82 | 10.25 | 40.08 |

The extracts of the invention at a concentration of 3.5 g/l and the commercial extract at a concentration of 0.31 g/l, have the same amount of punicalagin (92 mg/l) but the concentration of ellagic acid in the dry extracts according to the present invention is 1274 mg/ml, therefore 3.8 times the concentration of ellagic acid.

Ellagic acid and urolithin concentration over 48 hours was assessed by ProDigest Colon-on-a-plate ^{®} (COAP) technology.

### COAP technology

This is a miniaturised technology of the 'short-term in batch' version of the fermentation model, which has proven its ability not only to provide a detailed understanding of the interaction between a tested product and the human gut microbiota, but also the direct or indirect effects on host health.

This technology allows working with volumes an order of magnitude lower than those typically used in short-term fermentation batches.

Each well of the COAP platform functions as a mini-reactor in which the ability of specific substrates to modulate the composition of the bacterial community and its functioning is evaluated.

### Storage of faecal samples

Faeces samples from eight healthy adult donors were collected and processed within 48h. During cryopreservation, faeces samples were stored in an anaerobic container at refrigerator temperature. Prior to cryopreservation, faecal suspensions were prepared under anaerobic conditions and mixed with an in-house optimised cryoprotectant, i.e. a modified version of the cryoprotectant developed by Hoefman et al. (2013) [Efficient cryopreservation protocol allows accessibility of a wide range of ammonia-oxidising bacteria for the scientific community." Res Microbiol 164(4): 288-92]. The faecal suspensions obtained were rapidly frozen and then stored at -80 °C (cryostock) in anaerobic atmosphere 20 for long-term storage. It was ensured that each suspension underwent only one freeze-thaw cycle before being introduced into a reactor. Indeed, bacterial membranes can become damaged after repeated freezing cycles, resulting in a loss of function and ultimately viability. These actions ensure optimal preservation of the activity and composition of the cell community. Shortly before the experiment, an aliquot was thawed and immediately added to the reactors.

### COLON ON A PLATE^{®}

This simulation model allows the simultaneous testing of several conditions, with great benefit to reproducibility, as the same physical conditions are guaranteed for all simulated conditions. A COAP fermentation typically consists of administration of a single dose of a test compound to wells mimicking the colon environment, using a representative conventional nutritional medium and a bacterial inoculum from a human donor as the microbial source.

At the beginning of the experiment, COAP reactors were filled with a conventional carbohydrate-depleted nutritional medium representative for the colon environment (containing colonic basal nutrients, including peptone, yeast extract, mucin and L-cysteine). The test products were added from aqueous stock solutions to the respective reactors in the concentrations indicated in steps 1, 2, and 3 above. Finally, 10% (v/v) of a cryopreserved faecal inoculum containing 7.5% (w/v) faecal material from a healthy human donor was added to each reactor and served as the 10 microbial source. The final volume in the reactors was 28 ml.

The incubation temperature was 37°C and incubation was conducted under continuous gentle agitation (90 rpm) and an anaerobic atmosphere for 48h. Each experimental condition was tested on eight different donors to account for inter-individual differences, resulting in 24 reactors (1 treatment, one negative control and one competitor product as stated in 1. 2 and 3 above).

### Study endpoints

The Applicant has therefore surprisingly found that the combination of a dried pomegranate extract and Inulin has a synergistic action in improving intestinal homeostasis and in promoting the metabolism of polyphenols by the intestinal microbiota.

The samples were collected at baseline (0h) and 48h after the experimental start, and analysed for ellagic acid and urolithin A at baseline and after 48h.

### • Conversion of ellagic acid to urolithin A.

During digestion by the intestinal microbiota, ellagitannins (present in various edible plants including berries, pomegranate and nuts) are converted into ellagic acid, which is further metabolized into various urolithins. After cleavage and decarboxylation of a lactone in the ellagic acid molecule, further metabolism occurs through a series of dehydroxylation reactions [Djedjibegovic J, Marjanovic A, Panieri E, Saso L. (2020). Ellagic Acid-Derived Urolithins as Modulators of Oxidative Stress. Oxid Med Cell Longev 2020;2020:5194508 ]. Urolithins are powerful modulators of oxidative stress and agents with potential anti-inflammatory, anti-proliferative and anti-ageing properties.

The content of ellagic acid and urolithin A in the samples was analysed with high performance liquid chromatography coupled with a diode array-based detector (HPLC-DAD).

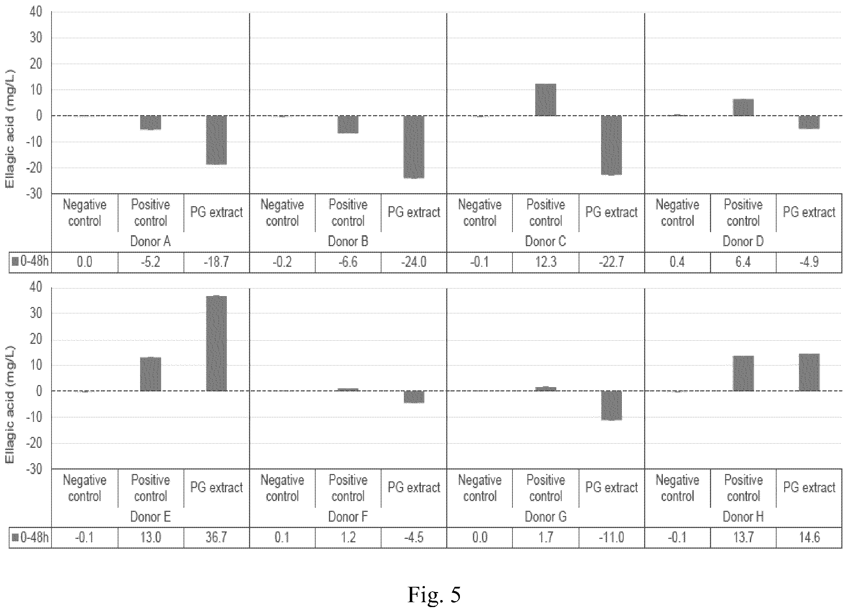

The results obtained are shown in figures 5- and 6. As can be seen in figure 5, the faecal inocula of 6 out of 8 subjects (A,B,C,D, F, G) treated with the composition of the invention are able to produce and consume ellagic acid with respect to the inocula treated with the commercial extract, consuming the ellagic acid faster than producing it from the relative punicalagin, when compared to the inocula of the same patients, but treated instead with the commercial extract, despite the concentration of ellagic acid in the extract of the invention being 3.8 times higher with respect to the concentration of ellagic acid in the commercial extract.

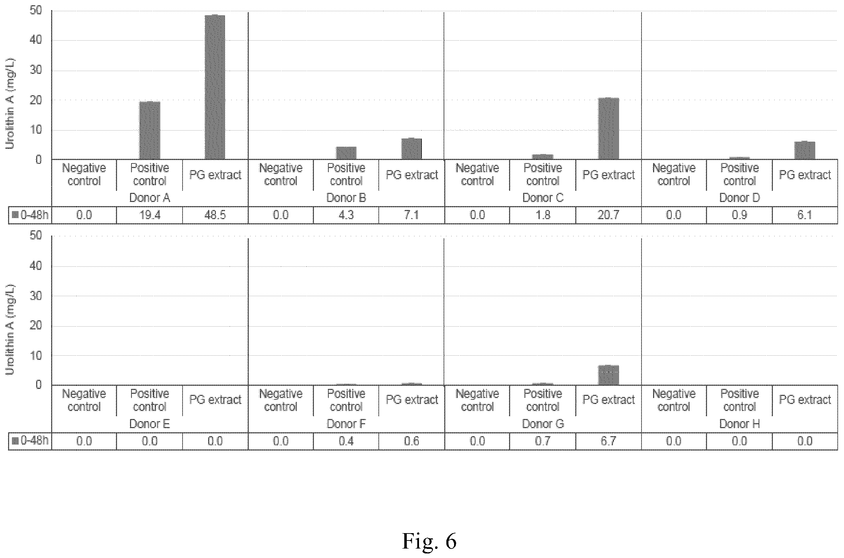

As can be seen in figure 6, the concentration of urolithin A, produced after 48 hours, was also much higher when the faecal inocula of the same subjects were administered the extract of the invention, with respect to the data obtained by administering the commercial extract.

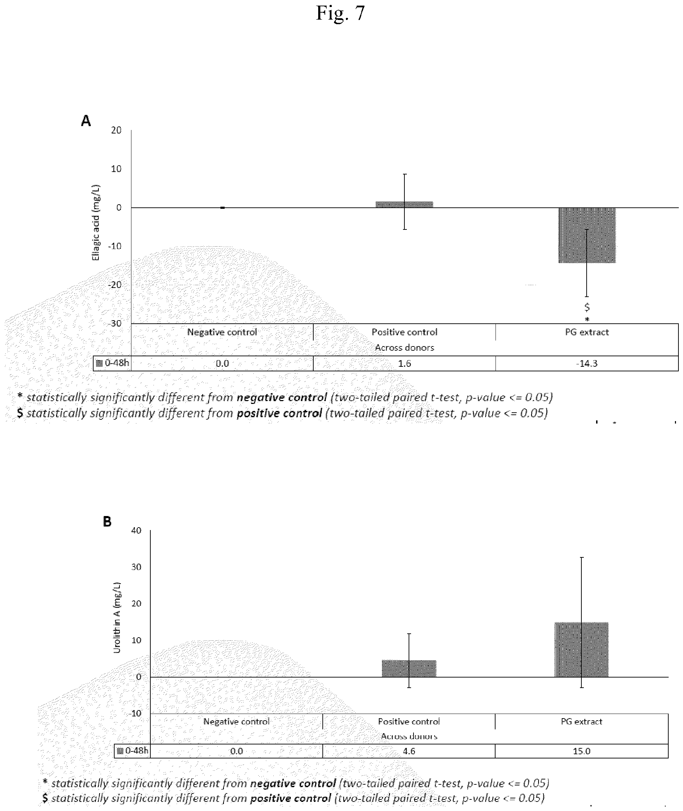

The results obtained with the six subjects able to convert ellagic acid and punicalagin to urolithin A were grouped into two graphs A and B of figures 7A and 7B, to obtain a statistical mean.

In the graph of Figure 7A it can be seen that, when the faecal inoculum is treated with the extract of the invention, the consumption of ellagic acid is higher than its production from punicalagin, while in the case of the commercial extract (positive control) it is not, since there is an accumulation of ellagic acid, albeit slight (+1.6). The value found with the extract of the invention is also statistically different with respect to the negative and positive control (p≤0.05).

Finally, with regard to figure 7B related to the production of urolithin A, although the result is not statistically significant, a clear increase in the production of urolithin A is however observed with respect to both the negative control and the positive control. To our knowledge, this is the first time that inulin has been used both as a technological support for the production of a pomegranate extract and for its effectiveness as an active prebiotic ingredient. Its inclusion in the granules makes its use even more interesting due to its double action for the purposes of having a stable extract but also with a greater bioavailability of pomegranate extract.

Pomegranate extract stabilized with Inulin (25%) increases intestinal production of urolithin A, with the same amount of punicalagin administered, and acts positively on mitochondrial metabolism.

## Claims

1. Powder and/or granular composition A) based on pomegranate extract comprising:
a) a dried pomegranate extract comprising punicalagin A and B, ellagic acid and/or derivatives thereof;
b) a powder comprising or consisting of Inulin
wherein:
- the content in total punicalagin A and B, ellagic acid and/or derivatives thereof is comprised between 4 and 15% by weight on the total weight of said powder and/or granular composition.
- the weight ratio between dried pomegranate extract / inulin is comprised between 1: 1 and 3: 1.

2. Powder and/or granular composition A) according to claim 1 wherein said powder composition b) comprises maltodextrin and/or silica.

3. Powder and/or granular composition A) according to claim 1 or 2 wherein in the powder mixture b) the maltodextrin is present in amounts comprised between 0 and 40% by weight on the total weight of said powder and/or granular composition A).

4. Powder and/or granular composition A) according to claim 2 or 3, wherein the amount of inulin is comprised between 20 and 30% of said powder and/or granular composition A).

5. Powder and/or granular composition A) according to any one of claims 2-4, wherein the amount of silica is comprised between 0 and 6% by weight on the total weight of said powder and/or granular composition A).

6. Powder and/or granular composition A) according to any one of claims 1-5, wherein said pomegranate extract is a dry extract selected from pericarp, endocarp, seeds, whole fruits, juice or relative mixtures, preferably it is pericarp extract.

7. Powder and/or granular composition A) according to any one of claims 1-6, wherein the content of total punicalagin A and B, ellagic acid and/or derivatives thereof is comprised between 4 and 10% by weight on the total weight of said powder and/or granular composition A).

8. Powder and/or granular composition according to claim 7 wherein the content of punicalagin A and B, ellagic acid and/or derivatives thereof is comprised between 6 and 8%, preferably is 7%.

9. Powder and/or granular composition A) according to any one of claims 1-8, wherein the weight ratio of dry pomegranate extract a) /inulin is comprised between 1.5:1 and 2.5: 1, preferably between 1.8:1 and 2.0:1.

10. Powder and/or granular composition A), according to any one of claims from 3 to 9, containing the dry extract a) in amounts comprised between 45 and 50% by weight, preferably 49% by weight, the maltodextrin in amounts comprised between 21 and 25% by weight, preferably 22% by weight, the inulin between 23 and 28% by weight, preferably 25% by weight, the silica in amounts comprised between 2 and 6% by weight, preferably 4% by weight, on the total weight of the powder and/or granular composition A).

11. Powder and/or granular composition A) according to any one of claims 1-10 wherein said powder and/or granular composition A) is obtained by drying a mixture A-1) comprising:
a-1) a soft pomegranate extract; and b) a powder comprising or consisting of inulin.

12. Process for preparing the powder composition A) according to claim 11, wherein said drying comprises the following steps:
I) provision of a soft extract a-1);
II) preparation of an aqueous suspension consisting of the soft extract a-1) and of the powder mixture b) comprising or consisting of inulin:
III) drying of the aqueous suspension, by evaporation of the solvent.

13. Process for preparing the granular composition A) according to claim 11, comprising the following steps:
i) the soft pomegranate extract a-1) is prepared;
ii) the powder b) comprising or consisting of inulin is poured into a fluid bed granulator and mixed
iii) in said fluid bed granulator it is granulated the soft extract a-1) prepared in step i) on the powder b) comprising or consisting of inulin prepared in the previous step ii);
iv) the granulate obtained in step iii) is discharged.

14. Process according to claim 12 or 13, for preparing the powder mixture A), wherein step I) or step i) of preparing the soft extract comprises the following steps:
1. provision of at least one fresh and/or dried part e) of the pomegranate selected from pericarp, endocarp, seeds, whole fruit and juice
2. mixing of at least a part e) of the pomegranate with a hydroalcoholic solvent f), preferably ethanol in water at concentrations comprised between 50% and 70% w/w, wherein the weight ratio e):f) is comprised in the range from 1:1 to 1:25, preferably from 1:3 to 1:20, more preferably from 1:6 to 1:15, said mixing being followed by a heating under stirring at a temperature comprised in the range from 30°C to 90°C, preferably from 40°C to 80°C, more preferably from 50°C to 70°C
3. extraction of the suspension of step 2) until complete exhaustion of biomass
4. separation of the solid material from the liquid phase by at least one filtration or centrifugation.
5. concentration by vacuum evaporation of the filtered liquid coming from step 4) until obtaining a soft extract having a concentration of dry material in water comprised between 25% and 75%;
6. eventual pasteurisation of the concentrated product coming from step 5.

15. Oral formulations comprising a powder and/or granular composition A) according to any one of claims 1-11 in combination with suitable excipients or diluents.

16. Oral formulations according to claim 15, in the form of a supplement, nutraceutical product, or medicament.

17. Oral formulations according to claim 15 or 16, comprising at least one further component selected from the group consisting of: manganese, purified fish oil, quercetin, zinc, pterostilbene, copper, citicoline, resveratrol, carnosine, L-carnitine, Hydroxymethylbutyrate, Coenzyme Q10, group B, C, D, E Vitamins, organic and/or inorganic Salts of magnesium, of selenium.

18. Powder and/or granular composition A) according to any one of claims 1-11, for use in the prevention of damage from ageing.

19. Powder and/or granular composition A) for use according to claim 18, wherein said damage from ageing involves different tissues and organs such as muscle, heart, brain, joints, immune system, liver, pancreas and kidney.

20. Powder and/or granular composition A) according to any one of claims 1-11, for use in improving intestinal homeostasis and/or in promoting the metabolism of polyphenols by the intestinal microbiota.
